# EUROPEAN PATENT SPECIFICATION

(11) **EP 2 838 528 B1**
(45) Date of publication and mention of the grant of the patent: **06.08.2025**
(21) Application number: 13868008.7
(22) Date of filing: 14.05.2013
(51) Int. Cl.: A61K 31/353, A61K 36/185, C07D 311/62, C07D 407/10, A61P 1/16, A61P 3/10, A61P 9/00, A61P 9/10, A61P 39/06, A61P 43/00, A61P 13/12

(54) **METHODS FOR INHIBITION OF SHC-1/P66 TO COMBAT AGING-RELATED DISEASES**
VERFAHREN ZUR HEMMUNG VON SHC-1/P66 ZUR BEKÄMPFUNG ALTERSBEDINGTER ERKRANKUNGEN
PROCÉDÉS D'INHIBITION DE SHC-1/P66 POUR COMBATTRE DES MALADIES LIÉES AU VIEILLISSEMENT

(30) Priority: 26.12.2012 US 201213727387
(43) Date of publication of application: 25.02.2015
(73) Proprietor: BELX BIO-PHARMACEUTICAL (TAIWAN) CORPORATION, Taipei City 114 (TW)
(72) Inventor: CHANG, Shau-Feng, Hsinchu City 30069 (TW); MA, Chun-Hsien, Hsinchu City 300 (TW); YANG, Kuo-Yi, Hsinchu City 300 (TW)
(74) Representative: Fuchs Patentanwälte Partnerschaft mbB
(86) International application number: PCT/CN2013/075599
(87) International publication number: WO 2014/101366

(56) References cited:
- EP-A1- 0 815 857
- EP-A1- 1 256 335
- EP-A1- 1 388 343
- EP-A1- 1 676 572
- EP-B1- 0 815 857
- EP-B1- 1 388 343
- WO-A1-00/56886
- WO-A1-00/78326
- WO-A1-01/05397
- WO-A2-2012/163588
- CA-A1- 2 830 616
- CA-C- 2 830 616
- CN-A- 1 443 533
- CN-A- 101 125 139
- CN-A- 101 822 372
- CN-A- 102 688 230
- CN-A- 102 688 501
- GB-A- 2 435 166
- JP-A- 2011 178 728
- BEECHER G R: "Proanthocyanidins: Biological activities associated with human health", PHARMACEUTICAL BIOLOGY, SWETS AND ZEITLINGER, LISSE, NL, vol. 42, no. SUPPL, 1 January 2004 (2004-01-01), pages 2 - 20, XP008101781, ISSN: 1388-0209, DOI: 10.1080/13880200490893474
- CTUAN Q ET AL.: "Study progress of proanthocyanidin in medicament", CHINESE JOURNAL OF CLINICAL RATIONAL DRUG USE, vol. 5, no. 2A, February 2012 (2012-02-01), pages 174 - 175, XP008175657
- TU XIAN-YU ET AL.: "Effects of OPC on Reducing Blood Glucose Diabetes Induced by STZ in Mice", JOURNAL OF YANGTZE UNIVERSITY( NATURAL SCIENCE EDITION)MEDICINE V, vol. 3, no. 4, December 2006 (2006-12-01), pages 247 - 249, XP008175335

## Description

### BACKGROUND

### (1) Technical Field

The technical field relates to the methods for inhibition of SHC-1/p66 to treat one or more symptoms of a SHC-1/p66-related disease.

### (2) Description of related art.

The median age of the world's population is increasing because of a decline in infertility and a 20-year increase in the average life span during the second half of the 20th century. (*See e.g.,* Centers for Disease Control and Prevention, Morbidity and Mortality Weekly Report, February 13, 2003, 52(06): 101-106). These factors, combined with elevated fertility in many countries during the 2 decades after World War II *(i.e.,* the "Baby Boom"), will result in increased numbers of persons aged >65 years during 2010 - 2030. Worldwide, the average life span is expected to extend another 10 years by 2050. The growing number of older adults increases demands on the public health system and on medical and social services. Chronic diseases, which affect older adults disproportionately, contribute to disability, diminish quality of life, and increased health- and long-term-care costs.

Research has been undertaken to determine a connection between the symptoms of aging and the biochemical and neurological basis of those symptoms. In particular, several biological pathways have been determined to prolong life span and protect from a variety of aging-associated diseases (Trinei et al., "P66Shc Signals to Age" Aging (2009) v. 1(6) pages 503-510). The presently described methods are directed to the biochemical and neurological basis for the symptoms of aging. In particular, the presently described methods show a positive effect on the SHC-1/p66 pathway, the number of reactive oxygen species (ROS) and thereby oxidative stress, liver function and pathology, and in motor control.

### SHC-1/p66 and Aging

It has been reported that mice lacking the 66kD isoform of the SHC (Src Homology and Collagen) protein family lived 30% longer than p66- proficient littermates. p66KO mice are long lived and appear, phenotypically normal, fertile, and healthy (Migliaccio et al., "The p66shc adaptor protein controls oxidative stress response and the life span in mammals. Nature. 1999; 402:309-313; see also Alam et al., Endocr. Relat Cancer. 2009 March ; 16(1): 1.) SHC proteins are known as adapter molecules, *i.e.* signaling components related to the assembly of macro-Research Perspective molecular complexes downstream of activated growth factor receptors (RTKs). A role for SHCs in insulin signaling has also been reported (Giorgetti et al. "Involvement of Src homology/collagen (SHC) proteins in signaling through the insulin receptor and the insulin-like-growth-factor-I-receptor." Eur. J. Biochem. 1994; 223:195-202). Thus, the p66KO mouse is one of the first mammalian example(s) of extended longevity by genetic attenuation of insulin/IGF signaling.

Instead, the linkage between p66 and longevity took an unexpected direction, becoming one of the strongest arguments in support of the Harman's "free radical theory of aging" (Harman, D., "A biologic clock: the mitochondria?" Journal of the American Geriatrics Society 1972; 20:145-147.): in fact, p66- deficient mice and cells present remarkably reduced levels of ROS and increased resistance to oxidative stress.

p66shc has a function completely distinct from that of the other SHC proteins: it was found that, in response to a number to prooxidant and apoptogenic stimuli, p66shc translocates to mitochondria, where it directly generates reactive oxygen species.

At molecular level, the isoforms of SHC, p66^{Shc}, p52^{Shc} and p46^{Shc}, largely share the same amino acid sequence at the C-terminus including the Src homologous type two domain (SH2), phosphotyrosine binding domain (PTB) responsible for the binding to phosphorylated tyrosine, and a region highly enriched in glycine and proline residues named collagen homologous (CH1) since its homology with collagen protein (Pelicci, G. et al., "A family of Shc related proteins with conserved PTB, CH1 and SH2 regions. Oncogene. 1996; 13:633-41). The peculiarity of p66Shc is an additional CH region (CH2) at its N-terminus (Pelicci, G. et al., "A novel transforming protein (SHC) with an SH2 domain is implicated in mitogenic signal transduction." Cell. 1992; 70: 93-104. Migliaccio, E. et al., "Opposite effects on the p52shc/p46shc and p66shc splicing isoforms on the EGF receptor-MAP kinase-fos signaling pathway. EMBO J. 1997; 16: 706-716).

### Reactive Oxygen Species, Cellular Stress, and Aging

Reactive oxygen species (ROS) superoxide and hydrogen peroxide perform important signaling functions in many physiological and pathophysiological processes. Cell senescence and organismal age are not exemptions. (Afanas'ev, Igor, Oxidative Medicine and Cellular Longevity, V.3 (2010), Issue 2, pages 77-85). ROS are potent inducers of apoptosis and execute the apoptotic program itself. (Giorgio et al. "Electron Transfer between Cytochrome c and p66Shc Generates Reactive Oxygen Species that Trigger Mitochondrial Apoptosis," Cell Vol. 122, 221-233, July 29, 2005).

In particular, SHC-1/p66 is stimulated by ROS signaling. Conversely, ROS are generated by SHC-1/p66.

As a result, SHC-1/p66 has been implicated in the molecular mechanisms underlying diabetes-induced oxidative stress and oxidant-dependent renal injury. (Menini et al., Diabetes 55:1642-1657 (2006)). Furthermore, SHC-1/p66 has been implicated in the diabetes related conditions such as high glucose associated endothelial dysfunction, atherognesis, diabetic nephropathy, and cardiomyopathy. (See Francia et al., J. Mol. Med. (2009) 87:885-891 and Berniakovich et al. JBC Vol. 283 No. 49, pp. 34283-34293, December 5, 2008)

### Aging and the Liver

An aged liver may show increased cellular degeneration compared to a young liver. Specifically, an aged liver cell may show impaired cell metabolism and specific changes in mitochondrial function and morphology. (Sastre et al. "Aging of the Liver: Age-Associated Mitrochondrial Damage in Intact Hepatocytes," Hepatology November. (1996) pp. 1199-1205 and Koch et al., "Role of the life span determinant P66shcA in ethanol-induced liver damage," Laboratory investigation (2008) 88, 750-760). For instance, gluconeogenesis and ketogenesis may be decreased, while mitochondrial size may increase. Hepatic cellular degeneration can be pathologically detected by evidence of ballooning or "foamy" cells, or steatosis (also called fatty change, fatty degeneration or adipose degeneration).

Ballooned cells are typically two to three times the size of adjacent hepatocytes and are characterized by a wispy cleared cytoplasm on H&E stained sections. They can be differentiated from adipocyte-like cells by their cytoplasm and nucleus; ballooned cells have their nucleus in the centre (unlike adipocyte-like cells which have it peripherally). Also, ballooned cells have (small) pyknotic nuclei or nuclei that are undergoing karyorrhexis, i.e. in the process of disintegrating. The cytoplasm of cells undergoing ballooning degeneration is wispy/cobweb-like, while adipocyte-like cells have a clear cytoplasm or a vacuolated one.

Steatosis is the process describing the abnormal retention of lipids within a cell. It reflects an impairment of the normal processes of synthesis and elimination of triglyceride fat. Steatosis has been connected to hepatocyte swelling caused by oxyradicals (Del Monte, "Swelling of hepatocytes injured by oxidative stress suggests pathological changes related to macromolecular crowding" Medical Hypotheses (2005) 64, 818-825).

### Aging effects on Motor Skills and Motor Deficits

With advanced age comes a decline in sensory motor control and functioning. These declines in fine motor control, gait and balance affect the ability of older adults to perform activities of daily living and maintain their independence (Yankner BA, Lu T, Loerch P. The aging brain. Annu Rev Pathol 2008;3:41-66.; Twohing, J. P. et al., "Age-dependent maintenance of motor control and corticostriatal innervation by death receptor 3." J. Neurosci. 2010. 30:3782-3792.) The causes of these motor deficits are multi-factorial, with central nervous system declines and changes in sensory receptors, muscles, and peripheral nerves playing a role.

Motor performance deficits for older adults appear to be due to dysfunction of the central and peripheral nervous systems as well as the neuromuscular system. (Seidler, R. *et al.,* "Motor control and aging: Links to age-related brain structural, functional, and biochemical effects."

Neuroscience and Biobehavioral Reviews 30 (2010) 721-733). Motor performance deficits include coordination difficulty (Seidler, RD et al., "Changes in multi-joint performance with age." Motor Control. 2002;6(1):19-31.), increased variability of movement (Contreras-Vidal et al., "Elderly subjects are impaired in spatial coordination in fine motor control," Acta Psychol (Amst). 1998 Nov;100(1-2):25-35; Darling et al., "Control of simple arm movements in elderly humans." Neurobiol Aging, 1989 Mar-Apr;10(2):149-57), slowing of movement (Diggles-Buckles V. "Age-related slowing. In: Stelmach GE, Homberg V, editors." Sensorimotor impairment in the elderly. Norwell, MA: Kluwer Academic; 1993.), and difficulties with balance and gait (Tang PF, Woollacott MH. "Balance control in the elderly." In: Bronstein AM, Brandt T, Woollacott MH, editors. Clinical disorders of balance, posture and gait. London: Arnold; 1996.) in comparison to young adults.

These deficits have a negative impact on the ability of older adults to perform functional activities of daily living. Gait and balance problems are of particular interest as falls are a major source of injury and morbidity in older adults: 20- 30% of older adults who fall suffer moderate to severe injuries that limit mobility and reduce quality of life. A pronounced increase in movement duration with age is seen on a variety of tasks. Movement slows with age by as much as 15-30% (cf. Diggles-Buckles, 1993). This slowing appears in part to be strategic in that older adults emphasize movement accuracy at the cost of movement speed. Slower information processing may also affect motor performance in a non-specific, global fashion due to an increase in neural noise and other synaptic changes.

Furthermore, a connection has been drawn between changes in gait patterns and aging. Specifically, in aged populations, gait is shortened and slowed down (Wolfson, L., et al., (1990) "Gait assessment in the elderly: A gait abnormality rating scale and its relation to falls." J. Gerontol. 45: M12-19). This recognized change in gait in humans has been correlated to an age-dependent gait change in rodents. (*See* Klapdor, K. et al., (1997) "A low-cost method to analyze footprint patterns." J. of Neuroscience Methods. 75: 49-54 and Hilber et al., (2001) "Motor skills and motor learning in lurcher mutant mice during aging." Neuroscience. 102:615-623).

EP 1 388 343 A1 relates to a method for preparing an extract of *Rosa multiflora* containing procyanidin B3 and comprising extraction of the underground part of *Rosa multiflora* with an organic solvent, followed by fractionation and purification of the extract by chromatography, and the EP 1 388 343 A1 also relates to use of the extract of *Rosa multiflora,* which prevents oxidation.

EP 1 256 335 A 1 relates to the field of cosmetics and relates to the use of oligomeric procyanolidines for the manufacture of skin treatment products, especially products that counteract skin aging.

EP 1 676 572 A1 relates to a therapeutic agent for the treatment of a disease in which the suppression of Th1 cytokine production is therapeutically effective and/or a disease in which the suppression of autoantibody production is therapeutically effective, a therapeutic agent for the eradicative treatment of these diseases, and an agent for inhibiting the progress of these diseases, comprising (i) a proanthocyanidin with a polymerization degree of at least 4 or (ii) a plant material extract containing a proanthocyanidin as an active ingredient.

WO 01/05397 A1 relates to the provision of procyanidin oligomers with biological activity against matrix metalloproteinase (MMP). The procyanidin oligomers can be isolated from the genus *Ulmus* and other plants and comprise trimeric through dodecameric procyanidin oligomers of flavan-3-ol monomer units. WO 01/05397 A1 relates to methods of using the procyanidin oligomer in treating tumor metastasis or invasion, rheumatoid arthritis, diabetes, corneal, epidermal, and gastric ulceration, skin wrinkling, periodontitis, osteoporosis; and in the promotion of wound and burn healing and other related maladies in which uncontrolled high levels of MMP are thought to play a role in the malady progress.

WO 00/78326 A1 relates to methods for preventing or treating the adverse physiological effects associated with cardiac disease by administering a purified proanthocyanidin composition to the person in an amount effective to prevent or reduce the adverse physiological effects.

WO 2012/163588 A2 relates to the provision of a topical or oral anti-ageing skin composition. Skin ageing, such as the appearance of fine lines, wrinkles or sagging, is a major age-related consumer issue. WO 2012/163588 A2 relates to topical or oral composition for delaying the visible signs of skin ageing is provided, the composition comprising curcumin and a composition containing as its main component proanthocyanidin oligomer to which a substance having a phloroglucinol ring structure or resorcinol ring structure has been bonded, which is obtainable by heating plant materials comprising proanthocyanidin polymer or aqueous extract thereof comprising pro-anthocyanidin polymer, with a substance having a phloroglucinol ring structure or resorcinol ring structure in an acidic aqueous solution.

EP 0 815 857 A1 relates to an antiobestic agent which has, in addition to the antiobestic effect, the effects of inhibiting saccharolytic digestive enzymes, suppressing an increase in blood sugar level, inhibiting the absorption of monosaccharides, adsorbing and excreting cholic acid, lowering cholesterol level and blood triglyceride level and, inhibiting lipase and is useful not only as an antiobestic agent but also as antilipotrophic, antihyperlipidemic, antiarteriosclerotic and antidiabetic agents. EP 0 815 857 A1 relates to an extract of tamarind seed coat being rich un procyanidin, which is the active ingredient in the antiobestic agent, exerts as such a potent antiobestic effect without being purified any more. The antiobestic agent serves as a saccharolytic digestive enzyme inhibitor, a hypoglycemic agent, a monosaccharide absorption inhibitor, a cholic acid adsorption/excretion agent, a cholesterol-lowering agent, a blood triglyceride level lowering agent and a lipase inhibitor and facilitates the production of foods, drinks and feeds showing these effects, thus contributing to the amelioration or prevention of diabetics or obesity in daily life.

CN 102 688 501 A relates to phospholipids of procyanidin B2. It relates to the material and its preparation methods and its main use for vascular dementia.

CN 101 125 139 A relates to a kind of proanthocyanidin B2 in the preparation of sugar control agents. It relates to the application of drugs for urinary diseases and vascular complications, by proanthocyanidin B2.

JP 2011/178728 A relates to an AMPK activator comprising dimeric to octameric proanthocyanidin as an active ingredient. Furthermore, it relates to a GLUT4 activator comprising proanthocyanidin as an active ingredient. JP 2011/178728 A relates also to uses as an antidiabetic agent, an anti-obesity agent, a visceral fat reducing agent or a visceral fat accumulation inhibitor each comprises these drugs.

CN 102 688 230 A relates to a procyanidin B2 known from traditional Chinese medicine which is used in preparation, prevention, diagnosis, detection, protection, treatment and research of vascular dementia disease and Alzheimer's disease, as well as pharmaceutical products related thereto.

The article "Proanthocyanidins: Biological Activities Associated with Human Health", Gary R. Beecher, Pharmaceutical Biology, Vol. 42, 2004, Pages 2-20 relates to a review of the beneficial health effects of proanthocyanidins.

CA 2 830 616 relates to a pharmaceutical composition for alleviating aggravation of hepatoma, improving hepatic function, improving hepatic fibrosis, improving hepatic cirrhosis, improving hepatitis and promoting injured liver regeneration. The pharmaceutical composition comprises an effective amount of procyanidin inlcuding BEL-X and a pharmaceutically acceptable carrier or salt.

WO 00/56886 discloses a method of reducing intracellular levels of reactive oxygen species (ROS) in a cell and a method of treatment of disease including myocardial infarction, stroke, premature ageing and diabetes, by using an agent capable of inhibiting the expression or activity of p66shc polypeptide, said agent being a nucleic acid molecule capable of reducing or preventing the expression of p66shc or an antibody binding domain capable of specifically binding to the p66shc polypeptide.

Chinese Journal of Clinical Rational Drug Use, 2012, 5, no. 2A, 174-175, discloses that proanthocyanidins exist in plants, such as grape seeds, and proanthocyanidins have therapeutical effects on anti-cancer, cardiovascular diseases and eye diseases.

Journal of Yangtze University, 2006, vol. 3, no. 4, 247-249, discloses the effect of oligomeric proanthocyanidin on diabetes on a mice model. It further discloses to obtain the oligomeric proanthocyanidin by extracting grape seeds, pine barks and red wine. It shows that the oligomeric proan-thocyanidin can reduce the blood sugar on the diabetes mice model.

None of the prior art documents disclose the use of the polymeric composition of the present application for use in the treatment of reperfusion injuries after ischemia.

### SUMMARY

The invention relates to a polymeric composition for use in treatment of one or more symptoms of a SHC-1/p66-related disease, wherein the SHC-1/p66-related disease is reperfusion injuries after ischemia, wherein the polymeric composition comprises monomer units having formula (I) as defined in the claims and/or a pharmaceutically acceptable salt or solvate thereof, wherein in Formula (I), each of R₁, R₂, R₃, R₇ and R₈ independently is H, R₄ is H or OH, R₅ is OH, and R₆ is OCH₃, and wherein the polymerized number of the monomer ranges from 2-30, and the average molecular weight of the polymer ranges from 600-10,000.

The disclosure relates to methods of treating one or more symptoms of a SHC-1/p66-related disease, inhibiting ROS generation or for the manufacture of a medicament in the above-mentioned treatment. In one embodiment, the present disclosure includes a method of treating one or more symptoms of a SHC-1/p66-dependent disease by administering a polymeric composition including monomer units having formula I and/or a pharmaceutically acceptable salt, solvate, or prodrug thereof: 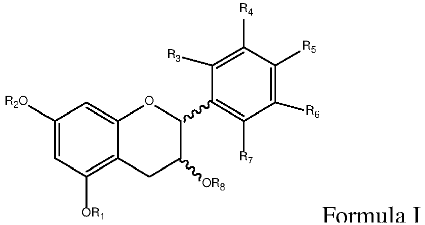 to a mammal in need thereof,
wherein in Formula I, each of R₁ and R₂, independently, is H, alkyl, or acyl; each of R₃, R₄, R₅, R₆, and R₇, independently, is H, OH, alkoxyl, or acyl; and R₈ is H or a saccharide moiety; and wherein the polymerized number of the monomer ranges from 2-30, and the average molecular weight of the polymer ranges from 600-10,000.

In one embodiment, the present disclosure includes use of a polymeric composition for the manufacture of a medicament to treat one or more symptoms of a SHC-1/p66-related disease selected from the group consisting of aging, diabetes, and reperfusion injuries after ischemia, in which the polymeric composition comprising monomer units having the formula I and/or a pharmaceutically acceptable salt, solvate, or prodrug thereof.

In one embodiment, the method according to the disclosure is directed towards treating one or more symptoms of a disease which is affected by expression or activity of SHC-1/p66. In a particular embodiment of the disclosure, "a SHC-1/p66-related disease" is one whose symptoms are ameliorated by decreasing the expression or activity of SHC-1/p66. Such diseases include aging, diabetes, and reperfusion injuries after ischemia. The present methods of the disclosure are directed to embodiments for treating one or more symptoms of aging, such as cellular degeneration, hepatocyte swelling, mitochondrial dysfunction, age-related motor deficits, and/or reduced stride length; one or more symptoms of diabetes such as high glucose associated endothelial dysfunction, atherogenesis, nephropathy, and/or cardiomyopathy; and one or more symptoms of reperfusion injuries after ischemia including the presence of increased reactive oxygen species and cell-death.

In one embodiment, the present methods include administration of a composition comprising BEL-X and/or a pharmaceutically acceptable salt, solvate, thereof. In one embodiment the composition is administered at a dose from 50 to 1,500 mg/kg/day.

A detailed description is given of the present disclosure and its specific embodiments below, with reference to the accompanying drawings.

### BRIEF DESCRIPTION OF THE DRAWINGS

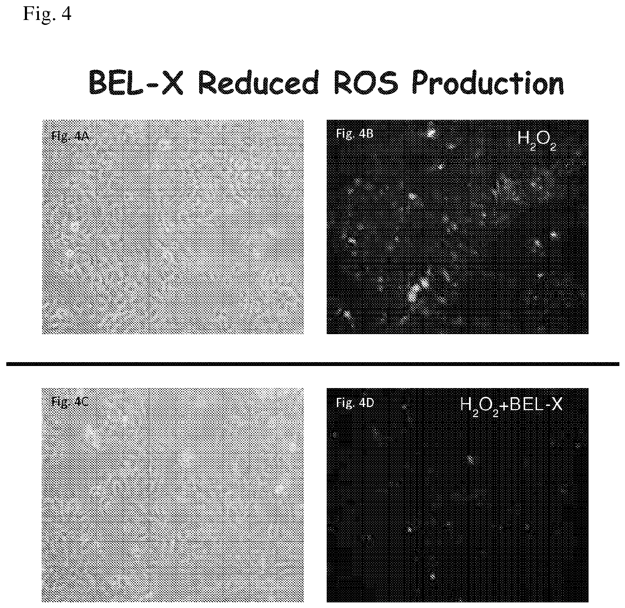

Fig. 1: Treatment of Herbal drug BEL-X effects on liver in aged mice according to the embodiment.
Figures 1A-C are hemotoxylin and eosin stains of slides of formalin fixed and paraffin embedded liver samples from young mice (2 months, Fig. 1A); old, untreated mice (20 months, Fig. 1B);
and old mice treated with BEL-X, p.o. at 1000 mg/kg/day for (20 months Fig. 1C) according to the embodiment.
Fig. 2: Detection of SHC-1/p66 expression by RT-qPCR. As shown in the graph, Bel-X reducted SHC-1/p66 expression according to the embodiment.
Fig. 3: Detection of the amount of SHC-1/p66 protein present in mouse liver tissue by using Western blotting according to the embodiment.
Fig. 4: Detection of ROS production after treatment of drug BEL-X in the cells according to the embodiment.
   Human hepatoma cells Huh7 were treated with H₂O₂ to induce ROS production (Fig 4A and 4C). The cells were treated with drug BEL- X after H₂O₂ induction (Fig 4B and 4D). Fig 4A and 4C showed a similar number of cells present on each slide observed by microscopy. Of those cells present, Fig. 4B shows a large production of ROS induced by H₂O₂ observed by immunofluorescences, while cells treated with drug BEL-X showed very little production of ROS indicted that BEL-X can reduce ROS protection and prevent the cells. (Fig. 4D).

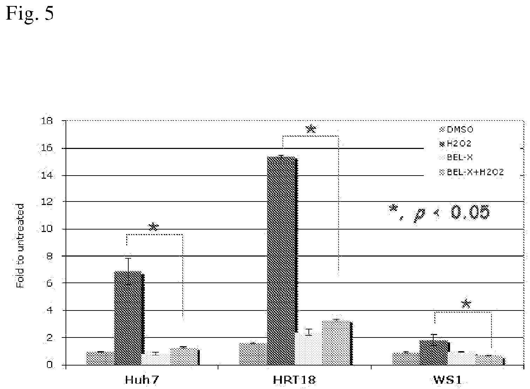

Fig. 5. Detection of the reduction of ROS in the cells according to the embodiment. The various types of cells including human hepatoma cell Huh7, human rectal cancer cell HRT-18 and human normal skin cell WS1 were tested. Notably, all tested cells showed that treated with BEL-X significantly less ROS generation after induction with H₂O₂.

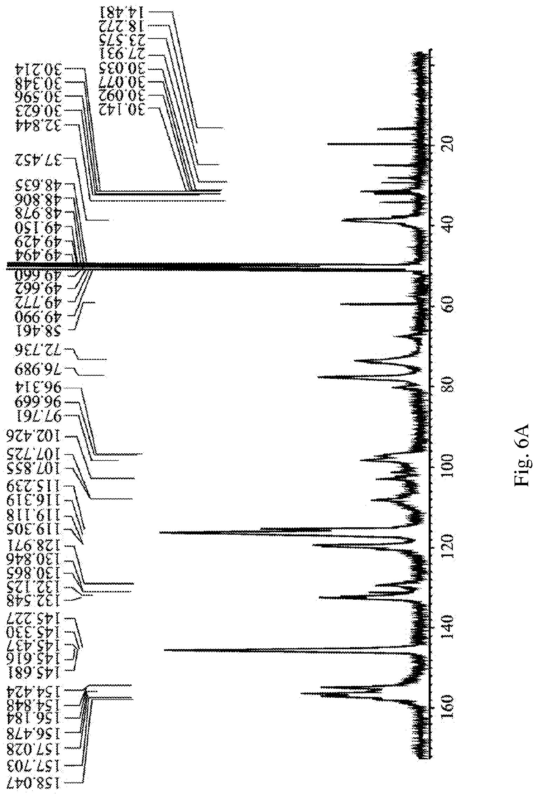

Fig. 6a-6c: show the ¹³C magnetic resonance spectroscopy results of purification of proanthocyanidins from *Boehmeria nivea* (L.) Gaud to obtain Bel-X according to the embodiment.

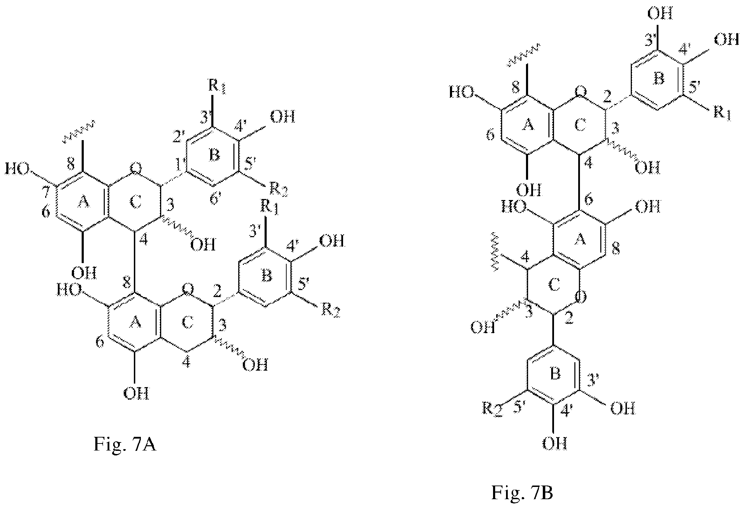

Figs. 7a-7b: show the bonding between two monomers of the proanthocyanidins according to the embodiment.

### DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

The embodiments of present compositions described herein include monomeric units of Formula I, or pharmaceutically acceptable salts, solvates, or prodrugs thereof: 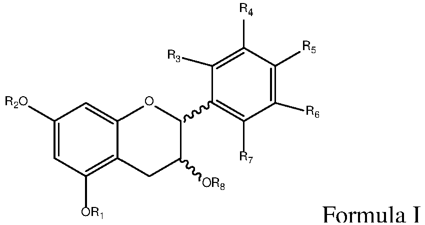 wherein each of R₁ and R₂, independently, is H, alkyl, or acyl; each of R₃, R₄, R₅, R₆, and R₇, independently, is H, OH, alkoxyl, or acyl; and R₈ is H or a saccharide moiety.

In one embodiment, the present disclosure includes a composition comprising a polymeric composition comprising monomer units having the formula I in the form of a nutrient, nutriceutical, health food, or supplement.

Referring to the above formula, the monomer units in the polymeric compound may have one or more of the following features: R₁ and R₂, independently, is H, each of R₃ and R₇ is H, and each of R₄, R₅, and R₆ is OH or alkoxyl, and R₈ is H.

In the polymeric compounds, monomer units may be covalently linked to each other via bonding between any two atoms of different monomer units, *e.g.,* C4-C8 bonding *(i.e.,* bonding formed between the C4 carbon of one monomer unit and the C8 carbon of another monomer unit), C4-C6 bonding *(i.e.,* bonding formed between the C4 carbon of one monomer unit and the C6 carbon of the other monomer unit), or C2-O7 *(i.e.,* bonding formed between the C2 carbon of one monomer unit and the O7 oxygen of the other monomer unit). In one example, all of the monomer units are covalently bonded to each other via C4-C8 bonding. In another example, all of the monomer units are covalently bonded to each other via C4-C6 bonding. Of note, the numbering of atoms of a cyclic compound is well known and commonly used in chemical nomenclatures. Shown below is the numbering of the atoms of the core structure of the polymeric compounds of Formula (I):

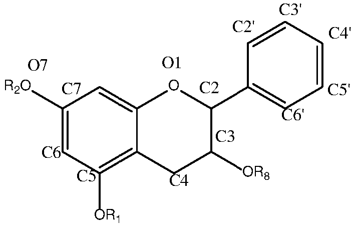

In one embodiment, the present compositions comprise compounds of Formula I with low oligomers; dimer, trimer, and tetramer. In other embodiments, the purified compositions comprise a mixture of monomeric units of Formula I with different degrees of polymerization.

The polymerized number of the monomer units may range from 2-30, more preferably, from 3-20. The average molecular weight is preferably from 600-10,000.

In one embodiment, the present compositions (including BEL-X) may contain mixtures of more than one monomer unit of Formula I. In one embodiment, the polymers may be homopolymers. In one embodiment the composition may comprise a mixture of different homopolymers. In one embodiment the polymers may be heteropolymers comprising multiple different monomer compounds falling within the scope of Formula I.

The "isolated preparation" refers to a composition containing one or more of the above-described polymeric compounds that has been partitioned from the natural source or the synthesis mixture.

The term "alkyl" refers to a straight or branched hydrocarbon, containing 1-10 carbon atoms. Examples of alkyl groups include methyl, ethyl, n-propyl, i-propyl, n-butyl, i-butyl, and t-butyl. The term "acyl" refers to a -C(O)-alkyl or -C(O)-aryl radical. Examples of acyl groups include - C(O)-CH₃ and -C(O)-ph. The term "alkoxy" refers to an -O-alkyl radical. Examples of alkoxy groups include: -OCH₃ and -OCH₂CH₃.

Alkyls mentioned herein can be either substituted or unsubstituted. Examples of a substituent include: halo, hydroxyl, amino, cyano, nitro, mercapto, alkoxycarbonyl, amido, carboxy, alkanesulfonyl, alkylcarbonyl, carbamido, carbamyl, carboxyl, thioureido, thiocyanato, sulfonamido, alkyl, alkenyl, alkynyl, alkyloxy, aryl, heteroaryl, cyclyl, heterocyclyl, in which alkyl, alkenyl, alkynyl, alkyloxy, aryl, heteroaryl cyclyl, and heterocyclyl are optionally further substituted with alkyl, aryl, heteroaryl, halogen, hydroxyl, amino, mercapto, cyano, or nitro.

The term "saccharide moiety" refers to a carbohydrate radical. It can be a radical of monosaccharide (e.g., allose, altrose, glucose, mannose, gulose, idose, galactose, talose, ribuose, psicose, fructose, sorbose, or tagatose), disaccharide (e.g., sucrose, lactulose, lactose, maltose, trehalose, or cellobiose), oligosaccharide (containing 3-10 monosaccharides), or polysaccharide (containing more than 10 monosaccharides).

Furthermore, the monomer of Formula I may comprise a flavonoid. The flavonoid may comprise catechin, epicatechin, epiafzetechin, gallocatechin, galloepicatechin, epigallocatechin, gallates, flavonols, flavandiols, leucocyanidins, or procynidins. In one embodiment, the monomer of the compound of Formula I may comprise flavan-3-ol or flavanones derivatives. Specific examples include: 3-flavanol, 3,4-flavanol, catechin ((2R,3S) and (2S,3R)) and, epicatechin ((2S,3S) and (2R,3R)), respectively.

The polymeric compounds described above include: the monomeric or polymeric compounds themselves, as well as their salts, prodrugs, and solvates, if applicable. A salt, for example, can be formed between an anion and a positively charged group (e.g., ammonium ion) on a polymeric compound. Suitable anions include: chloride, bromide, iodide, sulfate, nitrate, phosphate, citrate, methanesulfonate, trifluoroacetate, acetate, succinate, malate, tosylate, tartrate, fumurate, glutamate, glucuronate, lactate, glutarate, and maleate. Likewise, a salt can also be formed between a cation and a negatively charged group (*e.g.,* phenolate or carboxylate) on a polymeric compound. Suitable cations include sodium ion, potassium ion, magnesium ion, calcium ion, and an ammonium cation. The compounds may also be in prodrug and solvate form. Examples of prodrugs include esters and other pharmaceutically acceptable derivatives, which, upon administration to a subject, are capable of providing active compounds. A solvate refers to a complex formed between an active compound and a pharmaceutically acceptable solvent. Examples of pharmaceutically acceptable solvents include: water, ethanol, isopropanol, ethyl acetate, acetic acid, and ethanolamine.

The monomers of the polymeric compounds contain asymmetric centers. Thus, they can occur as racemates and racemic mixtures, single enantiomers, individual diastereomers, and diastereomeric mixtures. Such isomeric forms are contemplated. In one embodiment the monomer may comprise (R) or (S) optical isomers at the C4 position.

Another aspect of this disclosure relates to a method of inhibiting SHC-1 expression, inhibiting hepatocyte swelling, inhibiting ROS generation, or a method of reducing the occurrence or severity of age-related motor deficits, by administering to a subject in need thereof a pharmaceutical composition obtained by mixing a pharmaceutically acceptable carrier and the isolated preparation described above.

In one embodiment, the present disclosure includes use of a polymeric composition in for the manufacture of a medicament to inhibit SHC-1 expression, inhibit hepatocyte swelling, inhibit ROS generation, or reduce the occurrence or severity of age-related motor deficits, wherein the polymeric composition comprises monomer units having the formula I and/or a pharmaceutically acceptable salt, solvate or prodrug thereof.

Also within the scope of this disclosure is the use of the isolated preparation described above for methods to treat a SHC-1/p66-related disease, or for the manufacture of a medicament in the above-mentioned improvement/treatment/promotion.

Alternatively, the present compositions can be administered in food, as a nutrient, nutriceutical, health food, or supplement.

The details of many embodiments of the invention are set forth in the description below. Other features, objects, and advantages of the invention will be apparent from the description and the claims.

Polymeric compounds of Formula I, described above can be administered to a subject in need thereof to treat a SHC-1/p66-related disease and inhibit ROS generation.

The presently described compositions may be extracted from a plant, or modified, or synthesized by artificial means. In one embodiment, the plant may comprise a plant belonging to the Leguminosae, Crassulaceae, Combretaceae, Asclepiadaceae, Rosaceae, Lamiaceae, Polygonaceae, Ericaceae, Pinaceae, Vitaceae or Urticaceae family, and preferably is Boehmeria nivea (L.) Gaud belonging to the Urticaceae family. The part of the plant to be extracted may comprise a root, a stem, a leaf, and/or a fruit part.

Extraction methods are described in U.S. Patent Publication 2010/0168221 and U.S. 2011/0158933. The extract may be further partially purified or completely purified, optionally.

The terms "reduce" or "inhibit" includes a lowering of the severity or occurrence of a symptom. In one embodiment, reduction or inhibition is measured by a percentage when compared to either age-matched untreated controls and/or untreated young controls. In one embodiment the occurrence of symptoms may be reduced by at least 25%, 50%, or 75% when compared to age-matched controls. In one embodiment, the severity of the particular symptom may be reduced by at least 25%, 50%, or 75% to 99%, inclusive. A 100% reduction in the severity of symptoms would mean that the symptom is no longer present or detectable. In one embodiment, reduction or inhibition is measured by a "fold" increase in the occurrence or the severity of the symptom when compared to young mice. Preferably the fold increase is at least 25% in the occurrence or the severity of a symptom when compared to young mice.

The term "significant" is generally applied to statistically significant values. Statistical significance can be determined by generally understood methods.

The term significant means using a statistical method comparing treated and untreated groups or differently aged groups.

"Treating a SHC-1/p66-related disease" may mean treating one or more symptoms of a disease which is affected by expression or activity of SHC-1/p66. In particular, "a SHC-1/p66-related disease" is one whose symptoms are ameliorated by decreasing the expression or activity of SHC-1/p66. Such diseases include aging, diabetes, and reperfusion injuries after ischemia.

In particular, the present methods are directed to treating one or more symptoms of aging, such as cellular degeneration, hepatocyte swelling, mitochondrial dysfunction, age-related motor deficits, and/or reduced stride length; one or more symptoms of diabetes such as high glucose associated endothelial dysfunction, atherogenesis, nephropathy, and/or cardiomyopathy; and one or more symptoms of reperfusion injuries after ischemia including the presence of increased reactive oxygen species and cell-death.

The term "Age-Related Motor Deficits" may include a reduction in the speed of motor activity, reduction in stride length, or reduction in scope of motor activity. Reduction of age-related motor deficits can be a delay in the onset of age-related motor deficit or a reduction in the severity of the age-related motor deficit. In one embodiment, the onset of the age-related motor deficit is delayed in humans by at least 5 years, 10 years, 20 years, 30 years, 40 years, or 50 years in humans. In one embodiment, the reduction in the severity of the age-related motor deficit can be an improvement in the speed of the motor activity and/or the scope of the motor activity by at least 50%-100% (inclusive of each integer) when compared to the severity of the motor deficits of non-treated age-matched controls.

The subject may be an animal, more particularly a mammal, more particularly, a mouse, rat, rabbit, goat, or human.

### Dosage Form

To practice the method of the present invention, a composition containing one or more of the polymeric compounds described above, or their constituent monomers, can be administered parenterally, orally (*e.g.,* p.o.), nasally, rectally, topically, or buccally. The term "parenteral" as used herein refers to subcutaneous, intracutaneous, intravenous, intramuscular, intraarticular, intraarterial, intrasynovial, intrasternal, intrathecal, intralesional, or intracranial injection, as well as any suitable infusion technique.

A sterile injectable composition can be a solution or suspension in a non-toxic parenterally acceptable diluent or solvent, such as a solution in 1,3-butanediol. Among the acceptable vehicles and solvents that can be employed are mannitol and water. In addition, fixed oils are conventionally employed as a solvent or suspending medium (e.g., synthetic mono- or diglycerides). Fatty acid, such as oleic acid and its glyceride derivatives are useful in the preparation of injectables, as are natural pharmaceutically acceptable oils, such as olive oil or castor oil, especially in their polyoxyethylated versions. These oil solutions or suspensions can also contain a long chain alcohol diluent or dispersant, carboxymethyl cellulose, or similar dispersing agents. Other commonly used surfactants such as Tweens or Spans or other similar emulsifying agents or bioavailability enhancers which are commonly used in the manufacture of pharmaceutically acceptable solid, liquid, or other dosage forms can also be used for the purpose of formulation.

A composition for oral administration can be any orally acceptable dosage form including capsules, tablets, emulsions and aqueous suspensions, dispersions, and solutions. In the case of tablets, commonly used carriers include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavoring, or coloring agents can be added.

A nasal aerosol or inhalation composition can be prepared according to techniques well known in the art of pharmaceutical formulation. For example, such a composition can be prepared as a solution in saline, employing benzyl alcohol or other suitable preservatives, absorption promoters to enhance bioavailability, fluorocarbons, and/or other solubilizing or dispersing agents known in the art. A composition having one or more active compounds can also be administered in the form of suppositories for rectal administration.

The present compositions may be administered at a dose range from 50 to 1,500 mg/kg/day. In one embodiment, the present compositions are administered at a dose range of 250 to 1,000 mg/kg/day.

For increasing stride length a preferred dose range is: 50 -1000 mg/kg/day.

For decreasing hepatocyte swelling a preferred dos range is: 100-1000 mg/kg/day

The carrier in the pharmaceutical composition must be "acceptable" in the sense that it is compatible with the active ingredient of the composition (and preferably, capable of stabilizing the active ingredient) and not deleterious to the subject to be treated. One or more solubilizing agents can be utilized as pharmaceutical excipients for delivery of an active compound. Examples of other carriers include: colloidal silicon oxide, magnesium stearate, cellulose, sodium lauryl sulfate, and D&C Yellow # 10.

Alternatively, the present compositions can be administered in food, as a nutrient, nutriceutical, health food, or supplement.

The effects of a compound can be tested by an *in vitro* or *in vivo* assay. For example, compounds of this invention can be preliminarily screened by *in vitro* assays in which the compounds are tested for their bioactivity relating to oxidative stress. Compounds that demonstrate high efficacy in the preliminary screening can be further evaluated by *in vivo* methods well known in the art to evaluate their activity to reduce or inhibit expression or transcription of genes related to aging, such as SHC-1/p66.

### EXAMPLES

### Example 1: Production and Characterization of BEL-X

### Preparation of Boehmeria nivea (L.) Gaud Extract Having the Proanthocyanidins

### Method 1

The roots and stems of *Boehmeria nivea* (L.) Gaud were washed and dried in a natural environment. The dried *Boehmeria nivea* (L.) Gaud was cut into 5 mm thick slices and stored at 4 ° C. Then the stored *Boehmeria nivea* (L.) Gaud was ground by a grinder and then screened using a 20 mesh screen. The screened powder was taken and added into 95% ethanol (1:10, w/v), heated and refluxed for 2 hours (performed twice) and then cooled to room temperature. The heated and then cooled to room temperature extract solution was put into a centrifuge bag to be filtered by centrifuging. The filtered solution was concentrated by a vacuum evaporator at a temperature lower than 40 ° C., and then lyophilized by a lyophilizer. The lyophilized extract was a pharmaceutical composition containing an ingredient of proanthocyanidins.

### Method 2

The *Boehmeria nivea* (L.) Gaud stored at 4 ° C. in method 1 was ground by a grinder and then screened using a 20 mesh screen. The screened powder (less than 20 mesh) was taken and added into RO water (1:10, w/v), heated and refluxed for 2 hours (performed twice) and then cooled to room temperature. The heated and then cooled to room temperature extract solution was added into an ethanol aqueous solution (95-50%) and mixed. After the extract solution was cooled and precipitated, the upper layer solution was added into a centrifuge bag to be filtered by centrifuging. The filtered solution was concentrated by a vacuum evaporator at a temperature lower than 40° C., and then lyophilized by a lyophilizer. The lyophilized extract was a pharmaceutical composition containing an ingredient of proanthocyanidins.

### Purification of the Boehmeria nivea (L.) Gaud Extract

### Method 1

### Solvent Extracting-1

The *Boehmeria nivea* (L.) Gaud extract containing proanthocyanidins was added into a hexane (1:10 w/v), heated and refluxed for 6 hours to remove the lipid in the extract. The solid extract was dissolved in 70% methanol aqueous solution and/or 0.3% vitamin C solution and concentrated by a vacuum evaporator at a temperature lower than 40 ° C. to remove the solvent. Then, the extract was added into chloroform (extract: chloroform=1:1, v/v) and vortexed for 30 minutes (multiple extractions were performed). The water layer therefrom was added into ethyl acetate (extract: ethyl acetate=1:1, v/v) and vortexed for 30 minutes (multiple extractions were performed). The water layer therefrom was concentrated by a vacuum evaporator at a temperature lower than 40 ° C., and then lyophilized by a lyophilizer.

### Method 2

### Solvent Extracting-2

The *Boehmeria nivea* (L.) Gaud extract containing proanthocyanidins was dissolved in a water/ethanol solution removed ethanol by a vacuum evaporator at a temperature lower than 40 ° C., added into a hexane (1:10 v/v) and then vortexed for 30 minutes (multiple extractions were performed) to remove the lipid in the extract. The water layer therefrom was added into ethyl acetate (water layer: ethyl acetate=1:1, v/v) and vortexed for 30 minutes (multiple extractions were performed). The water layer therefrom was added into 1-butanol (1:1, v/v) and vortexed for 30 minutes (multiple extractions were performed). The water layer therefrom was concentrated by a vacuum evaporator at a temperature lower than 40 ° C., and then lyophilized by a lyophilizer.

### Method 3

### Gel Permeation Chromatography

The partial purified *Boehmeria nivea* (L.) Gaud extract containing proanthocyanidins in the method 1 was isolated by gel permeation chromatography (4 cm diameter x 45 cm long Sephadex LH-20) by using the solutions having different polarity ratios to elute, and remove impurities therein. 2.5 g of the partial purified substance was dissolved in 0.5 ml of 95% ethanol and placed into the gel permeation chromatography column and then continuously eluted with a serial of solvents. The eluted solutions eluted by different solvents were collected. The solvents were 300 ml of 95% ethanol, 300 ml of 95% ethanol/methanol (1/1. v/v), 300 ml of methanol, 300 ml of 50% methanol aqueous solution and 300 ml of 50% acetone aqueous solution, 300 ml of acetone respectively. Except for the eluted solution eluted by 300 ml of 95% ethanol, all other eluted solutions were concentrated by a vacuum evaporator at a temperature lower than 40 ° C., and then lyophilized by a lyophilizer. The lyophilized substance was stored at -20 ° C. for ready for use. The physical and chemical properties of the lyophilized *Boehmeria nivea* (L.) Gaud extract with partially purified and/or purified proanthocyanidins was analyzed. The lyophilized eluted substance had a partially purified and/or purified proanthocyanidins ingredient.

### ¹³C and ¹H Nuclear Magnetic Resonance Spectroscopy

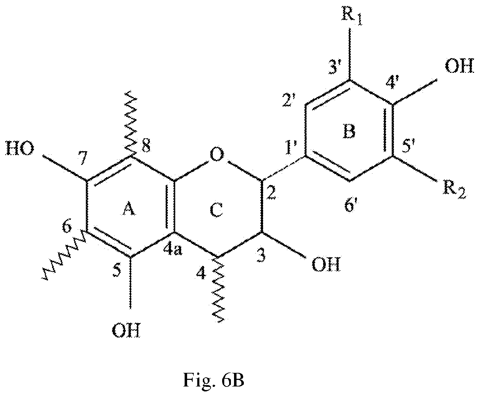

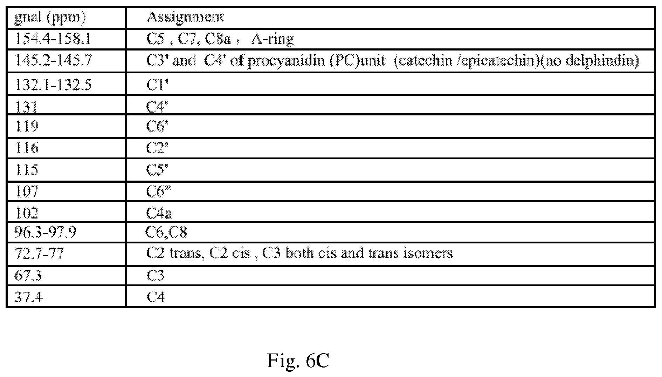

The purified proanthocyanidins sample was detected by ¹³C nuclear magnetic resonance spectrometry and ¹H nuclear magnetic resonance spectrometry. The ¹³C nuclear magnetic resonance spectroscopy results are shown as FIGS. 6a-6c, wherein at 145.2-145.7 ppm, there is only a peak of doublet-doublet and no other peak. Thus, the monomer had cyanidin but not delphindin, i.e. the B ring had three OH groups, which was identical with the EGA/MS analysis result. In FIG. 6, R₁=H or OH, and R₂=H, OH or OCH₃.

According the ¹³C nuclear magnetic resonance spectrum and the ¹H nuclear magnetic resonance spectrum, bonding between adjacent monomers of the proanthocyanidins mainly took place at the C4, C8 carbon-carbon bond. C4, C6 carbon-carbon bond unit and C2, C7 oxygen bond unit as shown in FIGS. 7a and 7b.

### Example 2: Effects of BEL-X

**Animal population:** Male and female C57BL/6 strain mice are used. There are 4 groups including (1) male mice not-treated (control), (2) male mice treated with BEL-X from age 9-20 months old, (3) female mice not-treated (control), (4) female mice treated with BEL-X from age 9-20 months old.

**Preparation and Dosage of Composition:** BEL-X from the method 3 above is dissolved in distilled water and 1000 mg/kg/day is delivered to the mice daily through p.o. administration using a feeding needle.

Mice are sacrificed at 20 months old. Liver tissues and sera are collected for pathologic and biological analysis.

### A. Effect of Bel-X on Aged Liver - reference example

Mouse body weight and liver weight are measured at mouse sacrifice. The livers are collected, fixed with formalin, and embedded in paraffin. Liver sections are subjected to hematoxylin and eosin staining. Hepatocyte swelling is evaluated by microscopy and a histopathologic evaluation of the fixed and stained liver sections.

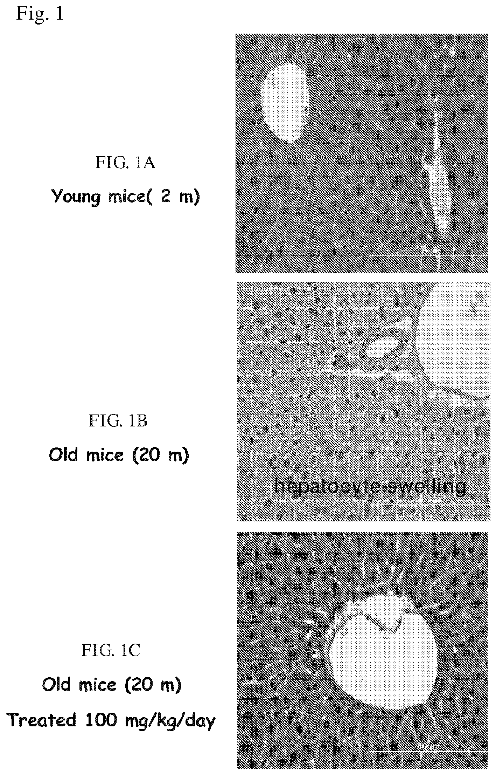

**Results** are shown in Fig. 1 A, B, and C. As shown by comparing the livers of young mice (2 months old) to old mice (20 months old), the livers of the old mice demonstrate hepatocyte swelling (compare Fig. 1A to Fig. 1B). Comparing the hepatocyte swelling of young mice to the level of hepatocyte swelling in old mice which are treated with Bel-X, the treated group shows minimal or no hepatocyte swelling; similar to the livers of young mice (see Fig. 1C). This is further demonstrated by the results in Table 1:

**Table 1:**

| **Groups** | **sex** | **Test No** | **Swelling and degeneration** | **Liver abnormal Ratio** |
|---|---|---|---|---|
| **control** | **male** | **12** | **9** | **75% (9/12)** |
| **BEL-X treated** | **male** | **12** | **3** | **25% (3/12)** |
| **control** | **female** | **10** | **10** | **100% (10/10)** |
| **BEL-X treated** | **female** | **12** | **3** | **25% (3/12)** |

Specifically, for male control (not treated) mice, twelve old mice are evaluated, nine of which show hepatocyte swelling and degeneration. Thus, the "liver abnormal ratio" is a percentage based on the total number of mice evaluated / the number of mice showing hepatocyte swelling. The higher the liver abnormal ratio, the more hepatocyte swelling was found.

Table 1 demonstrates that BEL-X treatment reduces the occurrence of hepatocyte swelling significantly in both male and female mouse populations, by 50-75%.

### B. Detection of SHC-1/p66 by RT qPCR

**Real-time RT-PCR:** Total RNA extraction is performed from frozen mouse livers as described as Trizol RNA isolation protocol. The cDNA synthesis uses random primers and the SuperScript II kit. The salt-free primer for target gene SHC1 isoform p66 forward primer, 5'-CGGAATGAGTCTCTGTCATCGCTGGA (SEQ ID NO: 1); reverse primer 5'-CGCCGCCTCCACTCAGCTTGTT (SEQ ID NO: 2) and for internal control house-keeping gene GAPDH forward primer, 5'- GAAGGTGAAGGTCGGAGT (SEQ ID NO: 3), reverse prime, r5'- GAAGATGGTGATGGGATTTC (SEQ ID NO: 4) are generated. To detect target gene expression levels in various liver tissues, one micro liter of total cDNA is added into 9 ml of real-time PCR mastermix (Roche Molecular Biochemicals) in the glass capillaries. A four-step experimental run protocol is used: (i) denaturation program (20 s at 95°C); (ii) the amplification and quantification program is repeated 60 times for SHC-1/p66 or 40 times for GAPDH (20 s at 95°C; 20 s at 62°C for SHC-1/p66 or 20 s at 60°C for GAPDH; 20 s at 72°C; 20 s at 82°C for SHC1/p66 and for GAPDH with a single fluorescence measurement); (iii) melting curve program (60-95°C with a heating rate of 0.1°C per s and a continuous fluorescence measurement); (iv) cooling program down to 40°C.

The RT-PCR products are calculated by C_{T} value using LightCycler Software 3.5 (Roche Molecular Biochemicals). The GADPH gene is used as reference. The relative SHC-1/p66 expression levels are analyzed by using the comparative C_{T} method (Schmittgen, T. D. & K.J. Livak. (2008) "Analyzing real-time PCR data by the comparative CT method." Nature Protocol. 3: 1101-1108.).

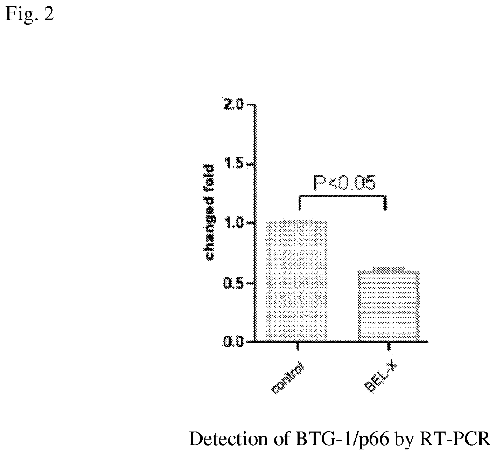

**Results:** As shown in Fig. 2, BEL-X reduces SHC-1/p66 expression significantly, when compared to the expression of SHC-1/p66 in mice not treated with BEL-X. Since p66- deficient mice and cells present reduced levels of ROS and increased resistance to oxidative stress, reduction of SHC-1/p66 expression also decreases ROS and reduces oxidative stress.

### C. Effect of Aging and BEL-X on SHC-1/p66 Protein - reference example

**Western blotting:** The liver tissues were added 10x volume of RIPA buffer and homogenized, and then removed the tissue debris by centrifugation, the solutions were used to separate proteins by SDS-PAGE. The proteins in SDS-PAGE were transferred onto PVDF membrane (Millipore), and incubated with specific anti-SHC1/p66 and GAPDH antibodies, respectively. The specific protein expression was detected and analyzed by using UVP Biospectrum.

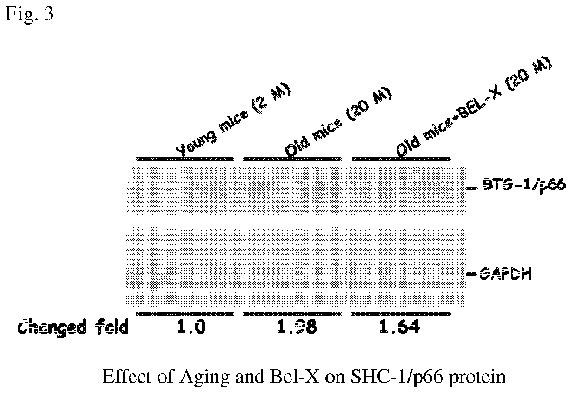

**Results:** Fig. 3 demonstrates that the SHC-1/p66 has nearly 2-fold higher protein concentration in old mice than in young mice. However, old mice treated with BEL-X showed significantly less SHC-1/p66 protein in the liver (having only about a 64% increase in protein concentration).

### Example 3: Motor Skills Evaluation - reference example

**Animals:** Male and female C57BL/6 strain mice are used. There are 4 groups including (1) male mice non-treated (control), (2) male mice treated with BEL-X from age 12-20 months old, (3) female mice non-treated (control), (4) female mice treated with BEL-X from age 12-20 months old. BEL-X is dissolved in distilled water and 250 mg/kg/day is delivered to the mice daily through p.o. administration using a feeding needle.

**Motor skill test:** The age-dependent changes in gait patterns of rodents have been compared to changes in the walking patterns of aging humans (Wolfson L, Whipple R, Amerman P, Tobin JN. (1990) Gait assessment in the elderly: A gait abnormality rating scale and its relation to falls. J Gerontol. 45:M12-19.) Therefore, a modified Gait test method is used (Klapdor, K. et al., (1997) A low-cost method to analyze footprint patterns. Journal of Neuroscience methods. 75:49-54; Hilber, P. and J. Caston. (2001) Motor skills and motor learning in lurcher mutant mice during aging. Neuroscience. 102:615-623.) to measure the stride width for differently aged mice. The paws of mice are stained with food coloring and the footprint is evaluated in 40x10 cm paper. The stride length is analyzed at least 3 times for each mouse.

**Results:** As shown by Table 2, old mice had significantly shorter strides than young mice, in both males and females. However, old mice treated with Bel-X had approximately the same stride length as young mice. Thus, treatment with Bel-X at 250 mg/kg/day helped older subjects retain their stride length.

**Table 2: Motor Skills Test**

| **group** | **age (months)** | **sex** | **No.** | **Gait Stride with (cm)** |
|---|---|---|---|---|
| **Mock** | **5** | **M** | **16** | **3.1 ± 0.1** |
| **Mock** | **20** | **M** | **16** | **2.6 ± 0.1*** |
| **BEL-X** | **20** | **M** | **16** | **3.2 ± 0.1^{# #}** |
| **mock** | **5** | **F** | **14** | **3.0 ± 0.1** |
| **mock** | **20** | **F** | **15** | **2.6 ± 0.1*** |
| **BEL-X** | **20** | **F** | **16** | **3.1 ± 0.1^{# #}** |

| | | | | |
|---|---|---|---|---|
| * p<0.01, compared with C57BL/6J mock-5M ; # p<0.01, ## p<0.001, compared with C57BL/6J mock-20M. | | | | |

Comparing a mouse life span to that of a human, it is clear that mice age more quickly than humans. According to reports from The Jackson Laboratory, mice at 3-6 months are approximately equivalent in age to a 20-30 year old human, and mice at 16-24 months are approximately equivalent in age to a 56-69 year old person. Accordingly, Bel-X treatment could be seen as providing a 56-69 year old with the stride length of a 20-30 year old.

### Example 4: Effect of Treatment on ROS Production

**Cell cultures:** Human hepatoma cells (Huh7) are maintained in MEM. Human rectal adenocarcinoma cells (HRT-18) and human skin fibroblastoid cells (WS1) are maintained in DMEM. The medium is supplemented with a 1% Penicillin/Streptomycin mixture and 1% non-essential amino acids, 1% GlutaMAX-I, 1 mM sodium pyruvate, and 10% fetal bovine serum. The cells are cultured at 37 ° C, 5% CO₂ incubator.

**ROS induction:** The cells are seeded in 24 wells plate and cultured for 24 hrs, and then 800 mM H₂O₂ is added to the culture medium for 1 hr to induce ROS production in the cells.

**BEL-X treatment:** To test drug BEL-X effects on ROS, the cells were treated with BEL-X for 24 hrs after H₂O₂ induction.

**Detection of ROS production:** 10 mM CM-H₂DCFDA is added to the cells and the cells are incubated at 37 ° C for 45 min. The cells are washed with PBS twice after the fluorescent probe incubation, the ROS production is observed by fluorescence microscopy directly or the ROS production is measured by fluorescence intensity using lysed cells and spectrophotometer.

**Results:** As shown by comparing Fig. 4B to Fig. 4D, human hepatoma Huh-7 cells induced with H₂O₂ to induce the generation of ROS show a large amount of ROS production (green fluorescence in Fig. 4B). However, cells which are induced to generate ROS, but which are also treated with Bel-X show very little ROS production (green fluorescence in Fig. 4D).

Further, as shown in Fig. 5, Bel-X significantly reduced ROS generation by H₂O₂ in all three cell types (human hepatoma cell Huh7, human rectal cancer cell HRT-18 and human normal skin cell WS1). Thus, comparing these experiments with the previous evaluations, Bel-X has an effect on SHC-1 which is in turn affected by the oxidative stress pathway. Accordingly, Bel-X reduces oxidative stress in a variety of cell types, such as liver, colon and skin cells.

## Claims

1. A polymeric composition for use in treatment of one or more symptoms of a SHC-1/p66-related disease, wherein the SHC-1/p66-related disease is reperfusion injuries after ischemia, wherein the polymeric composition comprises monomer units having formula (1) and/or a pharmaceutically acceptable salt or solvate thereof: 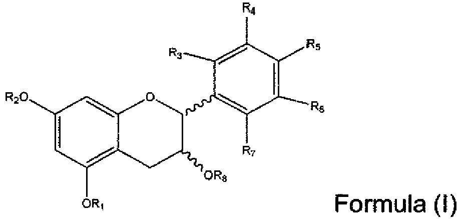 wherein in Formula (1), each of R₁, R₂, R₃, R₇ and R₈ independently is H, R₄ is H or OH, R₅ is OH, and R₆ is OCH₃, and wherein the polymerized number of the monomer ranges from 2-30, and the average molecular weight of the polymer ranges from 600-10,000.

2. A polymeric composition according to claim 1 for use according to claim 1, wherein the polymeric composition is administered parenterally, orally, nasally, rectally, topically, or buccally.

3. A polymeric composition according to claim 1 for use according to claim 1, wherein the polymeric composition is administered at a dose from 50 to 1,500 mg/kg/day.

4. A polymeric composition according to claim 1 for use according to claim 1, wherein the polymeric composition is in the form of a nutrient, nutriceutical, health food, or supplement.

## Patentansprüche

1. Polymere Zusammensetzung zur Verwendung bei der Behandlung von einem oder mehreren Symptomen einer mit SHC-1/p66 in Zusammenhang stehenden Erkrankung, wobei es sich bei der mit SHC-1/p66 in Zusammenhang stehenden Erkrankung um Reperfusionsverletzungen nach Ischämie handelt, wobei die polymere Zusammensetzung monomere Einheiten mit der Formel (I) und/oder ein pharmazeutisch verträgliches Salz oder Solvat davon umfasst: 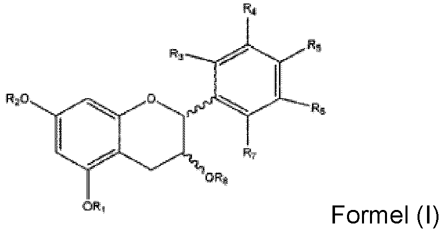
wobei in Formel (I) jedes von R₁, R₂, R₃, R₇ und R₈ unabhängig voneinander H ist, R₄ H oder OH ist, R₅ OH ist und R₆ OCH₃ ist,
und wobei die polymerisierte Anzahl des Monomers im Bereich von 2-30 liegt und das durchschnittliche Molekulargewicht des Polymers im Bereich von 600-10.000 liegt.

2. Polymere Zusammensetzung nach Anspruch 1 zur Verwendung nach Anspruch 1, wobei die polymere Zusammensetzung parenteral, oral, nasal, rektal, topisch oder bukkal verabreicht wird.

3. Polymere Zusammensetzung nach Anspruch 1 zur Verwendung nach Anspruch 1, wobei die polymere Zusammensetzung in einer Dosis von 50 bis 1.500 mg/kg/Tag verabreicht wird.

4. Polymere Zusammensetzung nach Anspruch 1 zur Verwendung nach Anspruch 1, wobei die polymere Zusammensetzung in Form eines Nährstoffs, Nutrazeutikums, Gesundheitsnahrungsmittels oder Nahrungsergänzungsmittels vorliegt.

## Revendications

1. Composition polymérique pour l'utilisation dans un traitement d'un ou plusieurs symptômes d'une maladie liée à SHC-1/p66, dans laquelle la maladie liée à SHC-1/p66 est une blessure de reperfusion après une ischémie, dans laquelle la composition polymérique comprend des unités monomères ayant la formule (I) et/ou un sel ou un solvant pharmaceutiquement acceptables de celle-ci : 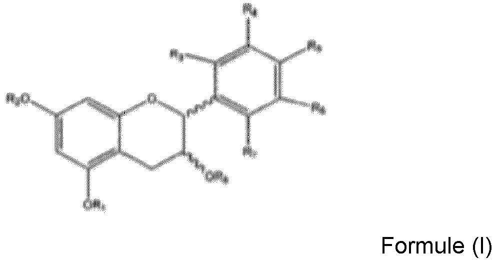
dans laquelle, dans la formule (I), chacun de R₁, R₂, R₃, R₇ et R₈ est H, R₄ est H ou OH, R₅ est OH et R₆ est OCH₃,
et dans laquelle l'indice de polymérisation du monomère est compris entre 2 et 30, et le poids moléculaire moyen du polymère est compris entre 600 et 10 000.

2. Composition polymérique selon la revendication 1 pour l'utilisation selon la revendication 1, dans laquelle la composition polymérique est administrée par voie parentérale, orale, nasale, rectale, topique ou buccale.

3. Composition polymérique selon la revendication 1 pour l'utilisation selon la revendication 1, dans laquelle la composition polymérique est administrée à une dose comprise entre 50 et 1 500 mg/kg/jour.

4. Composition polymérique selon la revendication 1 pour l'utilisation selon la revendication 1, dans laquelle la composition polymérique se présente sous la forme d'un nutriment, d'un nutriceutique, d'un aliment de santé ou d'un supplément, aliment de santé ou d'un supplément.
